# EUROPEAN PATENT SPECIFICATION

(11) **EP 3 866 610 B1**
(45) Date of publication and mention of the grant of the patent: **11.05.2022**
(21) Application number: 19801971.3
(22) Date of filing: 16.10.2019
(51) Int. Cl.: A23G 1/32, A23G 3/38, A61K 36/87, A61K 36/9066

(54) **CHOCOLATE MATRIX COMPRISING CURCUMIN AND RESVERATROL**
SCHOKOLADENMATRIX MIT CURCUMIN UND RESVERATROL
MATRICE DE CHOCOLAT COMPRENANT DE LA CURCUMINE ET DU RESVÉRATROL

(30) Priority: 16.10.2018 IT 201800009489
(43) Date of publication of application: 25.08.2021
(73) Proprietor: E.N.S. - European Nutritional Supplements S.r.l., 15057 Tortona (IT)
(72) Inventor: LEONE, Angelo, 15057 Tortona (IT); DAGLIA, Maria, 27100 Pavia (IT); BALDI, Alessandra, 15027 Pontestura (IT)
(74) Representative: Masala, Gian Tomaso
(86) International application number: PCT/IB2019/058818
(87) International publication number: WO 2020/079608

(56) References cited:
- WO-A1-2012/092916
- WO-A1-2018/087305
- CN-A- 104 824 303
- CN-A- 104 824 304
- DE-U1-202011 100 516
- US-A1- 2008 187 612
- US-A1- 2017 056 338
- US-B1- 6 773 744
- COUNET C ET AL: "Chocolate and cocoa: New sources of trans-resveratrol and trans-piceid", FOOD CHEMISTRY, ELSEVIER LTD, NL, vol. 98, no. 4, 1 January 2006 (2006-01-01), pages 649-657, XP027989531, ISSN: 0308-8146 [retrieved on 2006-01-01]

## Description

### FIELD OF THE INVENTION

The present invention relates to a chocolate matrix able to increase the oral bioavailability of plant derivatives such as resveratrol and curcumin, having beneficial effects in the maintenance of the normal physiological state, a nutraceutical product comprising said matrix, a nutraceutical kit for use as a food supplement or a functional food comprising said nutraceutical product, and a confectionery product comprising said matrix.

### BACKGROUND

The beneficial effects of some plant derivatives in the maintenance of the normal physiological state have been known for a long time and exploited, e.g. in traditional medicine and in the food sector.

Curcuminoids, for example, are a set of compounds made from curcuma (*Curcuma longa* L.) including curcumin, widely known for their antioxidant and anti-inflammatory properties. To date, curcuma has been widely used as an ingredient in food supplements, functional foods and common foods, whereas curcumin, which is one of the main components of curcuma to which the healthy properties of *Curcuma longa* are attributed, is also used in the food sector as an additive with a colouring function (E 100).

Among plant derivatives that have a beneficial action in the maintenance of the normal physiological state, 3,5,4'-trihydroxy-trans-stilbene is also known, commonly known as resveratrol, a polyphenol belonging to the class of stillbenes, naturally synthesised from different plant species and contained, for example, in grape skin, peanuts and mulberry, which performs an antioxidant, anti-inflammatory and blood thinning action. Because of these properties, resveratrol is now used as an ingredient in many food supplements.

However, it is also known that plant derivatives such as curcumin and resveratrol are characterised by poor solubility in aqueous means, e.g. fluids in the gastrointestinal tract (such as salivary fluid), and poor oral and gastrointestinal bioavailability. This, on one hand makes their action at physiological level less effective and, on the other hand, forces manufacturers to use larger quantities of said derivatives in the different products in which they are formulated, in order to guarantee the action thereof.

### SUMMARY OF THE INVENTION

The aim of the present invention is therefore that of providing a product that can increase the oral bioavailability of plant derivatives, such as resveratrol and curcumin, having beneficial effects in the maintenance of a normal physiological state.

According to the present invention, the Applicant has surprisingly found that these desired characteristics can be achieved by using a chocolate matrix comprising cocoa, ethanol and maltitol for the purpose of increasing the oral bioavailability of said plant derivatives.

Therefore, the present invention relates in a first aspect thereof to a chocolate matrix comprising cocoa, ethanol, maltitol and at least one plant derivative selected from the group consisting of: curcumin and resveratrol.

In fact, it has surprisingly been discovered that, thanks to the specific combination of components present in said matrix, the solubility of said plant derivatives in the salivary fluid increases, therefore increasing the bioavailability thereof.

Thanks to the specific combination of its components, the chocolate matrix according to the present invention therefore has a solubilising effect towards plant derivatives such as resveratrol and curcumin, in a physiologically significant aqueous means such as salivary fluid. This leads to an increase in the oral bioavailability of said plant derivatives, thus overcoming the limits set out above in relation to their efficacy on a physiological level, and allowing manufacturers to use smaller quantities thereof in the products in which they are used. This also implies a lower overuse of ingredients with high value and cost, greater possibility of developing targeted formulations, in order to guarantee the desired action.

In a further particularly preferred embodiment, the chocolate matrix according to the present invention comprises, with respect to the total weight of said matrix, from 40 to 65% by weight of cocoa, from 0.003 to 0.009% by weight of ethanol, from 30 to 55% by weight of maltitol, and from 1 to 2% by weight of at least one plant derivative selected from the group consisting of: curcumin and resveratrol.

The chocolate matrix according to the present invention is a product having a beneficial action in the maintenance of a normal physiological state, of particular value.

In a further aspect, the present invention therefore also relates to a nutraceutical product comprising the chocolate matrix according to the present invention and at least one nutraceutically suitable ingredient.

Thanks to the compositional characteristics of the chocolate matrix according to the present invention, the nutraceutical product according to the present invention in fact has many beneficial properties in the maintenance of the normal physiological state.

Said properties make the nutraceutical product according to the present invention particularly suitable for use as a food supplement or as a functional food.

In a further aspect, the present invention therefore also relates to a nutraceutical kit for use as a food supplement or as a functional food comprising at least one nutraceutical product according to the present invention and instructions for the user including information related to the use of the nutraceutical product.

As well as the actions at physiological level illustrated above, the chocolate matrix according to the present invention is a particularly valuable product also from a dietary point of view, particularly suitable for the confectionery sector.

In a further aspect, the present invention therefore also relates to a confectionery product comprising the chocolate matrix according to the present invention and at least one suitable food additive.

In a particularly preferred embodiment, the confectionery product according to the invention is a chocolate or a chocolate-based spreadable cream.

### DETAILED DESCRIPTION OF THE INVENTION

Therefore, the present invention relates in a first aspect thereof to a chocolate matrix comprising cocoa, ethanol, maltitol and at least one plant derivative selected from the group consisting of: curcumin and resveratrol.

The present invention can be presented in one or more of its aspects or one or more of the preferred characteristics reported below, which can be combined with one another as preferred according to the application requirements.

Within the context of the present description and following claims, all the numerical magnitudes indicating quantities, parameters, percentages, and so on are to be considered preceded in every circumstance by the term "about" unless indicated otherwise. Further, all the ranges of numerical magnitudes include all the possible combinations of maximum and minimum numerical values and all the possible intermediate ranges, as well as those indicated below.

Within the context of the present invention a specific combination of components has been identified which can increase the solubility of plant derivatives such as resveratrol and curcumin in a physiologically significant aqueous means such as salivary fluid, thus also increasing the oral bioavailability.

In fact, thanks to the specific combination of the components thereof, the chocolate matrix according to the present invention has a solubilising effect towards said plant derivatives in a physiologically suitable aqueous means, such as salivary fluid, thus promoting the absorption by the organism at oral level. This also leads to an increase in the oral bioavailability of said plant derivatives and enables the limits set out up to now in relation to their efficacy on a physiological level to be overcome, and smaller quantities thereof to be used in the products in which they are inserted. This also implies lower overuse of said derivatives as well as an increase in formulation terms, thanks to the possibility of developing formulations that aim to have a known dosage, in order to guarantee the desired action thereof.

In the present invention when the expression:
- "cocoa" is used it means a product derived from any part of the beans of the cocoa tree (*Theobroma cacao* L.) and any sub-species thereof, including its sugars, fats, amino acids, alkaloids and polyphenols. Typical examples of products deriving from said beans are cocoa bean powder the fatty substance obtained from cocoa beans or from parts of cocoa beans (cocoa butter);
- "nutraceutical product" is used it means a product comprising at least one molecule with beneficial properties in the maintenance of a normal physiological state, having an action at nutritional level and at physiological level; and
- "curcumin" is used it means a set of products of curcuma (*Curcuma longa* L.), comprising one or more curcuminoids selected from the group that consists of: diferuloylmethane (also known as curcumin I), p-hydroxycinnamoyl feruloyl methane (also commonly known as demethoxycurcumin or curcumin II), and p,p-di-hydroxydicinnamoylmethane (also commonly known as bisdemethoxycurcumin or curcumin III).

The chocolate matrix according to the present invention comprises cocoa.

In the chocolate matrix according to the present invention the cocoa can be contained as cocoa bean powder, cocoa butter, and mixtures thereof.

Preferably, the chocolate matrix according to the present invention comprises cocoa in an amount from 40 to 65% by weight, more preferably in an amount from 45 to 60% by weight, with respect to the total weight of the matrix.

The chocolate matrix according to the present invention comprises ethanol.

Preferably, the matrix according to the present invention comprises ethanol in an amount from 0.003 to 0.009 % by weight, more preferably from 0.004 to 0.008 % by weight, with respect to the total weight of said matrix.

Said amount of ethanol has in fact been shown to be optimal for increasing the solubility in salivary fluid of plant derivatives such as resveratrol and curcumin.

The chocolate matrix according to the present invention further comprises maltitol. Preferably, the matrix according to the present invention comprises maltitol in an amount from 30 to 55% by weight, more preferably from 35 to 50% by weight, with respect to the total weight of said matrix.

In an aqueous means such as salivary fluid, said quantity of maltitol has been shown to be ideal for allowing greater solubilisation of said plant derivatives.

In fact, said combination of components in said quantities has been shown to be optimal in order to increase the solubility of the at least one plant derivative in the salivary fluid, thus also allowing improved bioavailability.

Preferably, the chocolate matrix according to the present invention comprises the at least one plant derivative in an amount from 1 to 2% by weight, more preferably from 1.2 to 1.8% by weight, with respect to the total weight of said matrix.

Preferably, the at least one plant derivative comprises curcumin.

More preferably, the curcumin comprises from 70 to 80% by weight of diferuloylmethane.

More preferably, the curcumin comprises from 15 to 25% by weight of p-hydroxycinnamoyl feruloyl methane.

More preferably, the curcumin comprises from 2.5 to 6.5% by weight of p,p-di-hydroxydicinnamoylmethane.

More preferably, the curcumin comprises from 70 to 80% by weight of diferuloylmethane, 15 to 25% by weight of p-hydroxycinnamoyl feruloyl methane, from 2.5 to 6.5% by weight of p,p-di-hydroxydicinnamoylmethane.

Preferably, the at least one plant derivative comprises resveratrol and curcumin.

In a further particularly preferred embodiment, the chocolate matrix according to the present invention comprises, with respect to the total weight of said matrix, from 40 to 65% by weight of cocoa, from 0.003 to 0.009% by weight of ethanol, from 30 to 55% by weight of maltitol, and from 1 to 2% by weight of at least one plant derivative selected from the group consisting of: curcumin and resveratrol.

As well as the components described above, the chocolate matrix according to the present invention can advantageously comprise one or more further components such as, for example, sugars and/or polyols other than maltitol, flavourings.

The chocolate matrix according to the present invention can be prepared according to methods known for this purpose to a person skilled in the art of preparing chocolate. The at least one plant derivative can be added in any suitable step of the production of the chocolate matrix according to the present invention.

The chocolate matrix according to the present invention is a product having an action on a nutritional level and physiological level, of particular value.

In a further aspect, the present invention therefore also relates to a nutraceutical product comprising the chocolate matrix according to the present invention and at least one nutraceutically suitable ingredient.

The nutraceutical product according to the present invention can preferably be used as a food supplement or as a functional food, usable in a wide range of physiological conditions, also as a function of the type of plant derivative used.

Advantageously, for example, the nutraceutical product in which the plant derivative comprises curcumin has particularly high antioxidant and anti-inflammatory activity and in general improved with respect to the prior art, as a function of the greater solubility of the latter in salivary fluid.

Advantageously, when the plant derivative comprises resveratrol, the nutraceutical product has antioxidant, anti-inflammatory and blood thinning properties that are generally higher with respect to the prior art, thanks to the greater solubility of said plant derivative in salivary fluid.

The nutraceutical product according to the present invention can therefore advantageously be used as a food supplement or a functional food, preferably while maintaining a physiological state of health.

The nutraceutical product according to the present invention comprises at least one nutraceutically suitable ingredient such as, for example, dry elder extract (*Sambucus nigra* L.), Vitamin E, Lycopene, Vitamin A, Vitamin D3, folic acid.

The nutraceutical product according to the present invention can present in any form that makes it suitable for the final use, for example it can present in liquid, solid or gel form.

In a preferred embodiment, the nutraceutical product according to the present invention is in solid form.

Preferably, the nutraceutical product according to the present invention is prepared in the form of a capsule, granulate, pill, candy, tablet, paste, cream.

Thanks to the properties of the nutraceutical product according to the present invention, it can be used as a food supplement or as a functional food.

In a further aspect, the present invention therefore also relates to a nutraceutical kit for use as a food supplement or as a functional food comprising at least one nutraceutical product according to the present invention and instructions for the user including information related to the use of the nutraceutical product.

As well as the actions at nutritional and physiological level, the chocolate matrix according to the present invention is a particularly valuable product also from a dietary point of view, particularly suitable for the confectionery sector.

In a further aspect, the present invention therefore also relates to a confectionery product comprising the chocolate matrix according to the present invention and at least one suitable food additive.

The confectionery product according to the present invention can preferably be used in the confectionery sector, advantageously in the production of chocolates, spreadable creams, biscuits, cakes and puddings, confectionery, advantageously in the production of sweets and jams and in the production of ice-cream. The confectionery product is advantageously suitable for the administration of at least one plant derivative selected from the group that consists of: curcumin and resveratrol.

In a particularly preferred embodiment, the confectionery product according to the invention is a chocolate or a chocolate-based spreadable cream.

The confectionery product according to the present invention comprises at least one suitable food ingredient such as, for example, Vitamin E, Vitamin A, Vitamin D3.

### EXPERIMENTAL PART

The invention is now described by means of some examples to be considered for non-limiting illustrating purposes thereof.

### Example 1 - Evaluation of the solubility of curcumin and resveratrol

### Materials and Methods

### Materials

- Venchi dark chocolate, (100 g bar made by Venchi S.p.A. - Castelletto Stura, CN, Italy), comprising 60% by weight of cocoa;
- Maltitol powder (Venchi S.p.A. - Castelletto Stura, CN, Italy);
- Resveratrol in powder form (Ens S.r.l. - Tortona , AL, Italy);
- Curcumin, contained in *Curcuma longa* L. rhizome powder (Ens S.r.l. - Tortona , AL, Italy);
- Ethanol 96 ° Food Grade, (Sigma Aldrich - Milan, MI, Italy);
- KCI, KH₂PO₄, NaHCO₃, MgCl₂(H₂O)₆, (NH₄)₂CO₃, (Carlo Erba reagents S.r.l. - Milan, Italy);
- DMEM (Dulbecco's minimal essential medium) (Sigma Aldrich - Milan, Italy);
- PBS (Phosphate-buffered saline) (Sigma Aldrich - Milan, Italy);
- HaCaT (Human immortalized keratinocytes cell line) (Sigma Aldrich - Milan, Italy)

### Preparation of simulated salivary fluid (SSF)

The SSF was prepared following the instructions included in the article published by Minekus et al. in 2014 in the journal Food & Function (Minekus M, Alminger M, Alvito P, et al. A standardized static in vitro digestion method suitable for food - an international consensus. Food & Funct. 2014. 5:1113-1124). In detail, the SSF was prepared by mixing the following saline solutions:
KCI at the concentration of 15.1 mmol/l,
KH₂PO₄ at the concentration of 3.7 mmol/l,
NaHCO₃ at the concentration of 13.6 mmol/l,
MgCl₂(H₂O)₆ at the concentration of 0.15 mmol/l, and
(NH₄)₂CO₃ at the concentration of 0.06 mmol/l.

### HPLC-DAD chromatography method

The chromatography analyses were performed using a HPLC-DAD Agilent 1100 system (Agilent, Waldbronn, Germany), comprising a quaternary pump, self-sampler with thermostat equipped with a 100 µl injection loop, column thermostat, DAD Agilent 1100 detector (Agilent, Waldbronn, Germany). For the system control and data analysis the Chemstation software was used. The separation of the compounds was obtained with a Gemini C18 110A analytical column (150 mm × 2 mm; particle diameter 5 µm), connected to a suitable pre-column, both purchased from Phenomenex, Torrance, CA, USA. The mobile phase used comprised water acidified with 0.1% formic acid (eluent A) and methanol (eluent B), according to the elution gradient shown in Table 1 below.

**Table 1: Elution gradient**

| **Time (min)** | **Eluent A (% in volume)** | **Eluent B (% in volume)** |
|---|---|---|
| 0 | 90 | 10 |
| 40 | 0 | 100 |
| 50 | 0 | 100 |
| 57 | 90 | 10 |
| 62 | 90 | 10 |

The mobile phase flow rate was set to 0.3 ml/min, the injection volume to 5 µl, the temperature of the column to 25 °C and the temperature of the self-sampler was maintained at 4 °C. The chromatograms were purchased at two wavelengths at which the two compounds of interest had maximum absorption: 420 nm for curcumin and 320 nm for resveratrol.

Each sample as illustrated below was analysed in triplicate and the concentration of curcumin and resveratrol was calculated on the basis of two calibration curves previously constructed using the curcumin and resveratrol supplied as reference substance and dissolved in 96% ethanol. For processing the calibration curves, the curcumin and resveratrol solutions (prepared by dissolving an exactly weighed quantity of substance in 96% ethanol) were prepared and subjected to analysis in a concentration range, from 0.5 to 200 µg/ml .

### Example 1a - Evaluation of the solubility of curcumin and resveratrol in SSF

In order to evaluate the solubility of curcumin and resveratrol in SSF, 50 mg of curcumin and 50 mg of resveratrol were weighed in a 15 ml Falcon test tube; 6 ml of SSF were added to it. After mixing the aforementioned components, the sample was filtered and subjected to chromatography analysis, according to the HPLC-DAD chromatography method described above.

The results obtained through chromatography analysis highlighted the peaks attributable to curcumin and resveratrol, confirming the substantial insolubility of said compounds in SSF.

### Example 1b - Evaluation of the solubility of curcumin and resveratrol in SSF, in the presence of a chocolate matrix comprising cocoa, ethanol and maltitol

In order to evaluate the solubility of curcumin and resveratrol in SSF in the presence of a chocolate matrix comprising cocoa, ethanol and maltitol, two samples of about 3 grams were prepared, according to the following procedure: 2.8528 g of Venchi dark chocolate were dissolved at 50°C in bain-marie in two beakers, to which the quantities of maltitol, ethanol, curcumin and resveratrol indicated in Table 2 were added.

**Table 2: Quantity of maltitol, ethanol, curcumin and resveratrol used**

| **Substances** | **Sample 1** | **Sample 2** |
|---|---|---|
| Maltitol | 1.35 g | 1.35 g |
| Ethanol | 199.8 µL | 99.9 µL |
| Curcumin | 50 mg | 50 mg |
| Resveratrol | 50 mg | 50 mg |

After sufficiently mixing the samples at room temperature, they were placed at - 80°C for solidifying and subsequently an extraction was performed on each of them at room temperature, using 6 ml of SSF. The samples were filtered and subjected to chromatography analysis, according to the HPLC-DAD chromatography method described above in Example 1a. The results obtained are reported in Table 3.

**Table 3: Results obtained from the chromatography analysis of the two samples**

| | | **Curcumin** | **Resveratrol** |
|---|---|---|---|
| **SAMPLE 1** | Retention time | 32,816 | 21,720 |
| | Area of the chromatography peak | 91.1351 | 15970.1 |
| | Concentration (µg/mL) | 3.31 | 155.81 |
| **SAMPLE 2** | Retention time | 32.659 | 21.768 |
| | Area of the chromatography peak | 75.8888 | 8448.03 |
| | Concentration (µg/mL) | 3.16 | 82.47 |

For both samples, it was possible to highlight the solubilisation of the curcumin and the resveratrol in the presence of the chocolate matrix comprising cocoa, ethanol and maltitol.

Considering the two different concentrations of maltitol and ethanol tested, in the test conditions used, it was also possible to note that, the highest concentration of maltitol and ethanol corresponds to greater solubility of curcumin and resveratrol in the SSF.

### Example 2 - Evaluation of the oral bioavailability

### Example 2a - Evaluation of the oral bioavailability in vitro of curcumin and resveratrol in vitro in HaCat cells

In order to evaluate the oral bioavailability of curcumin and resveratrol a simulative test was performed *in vitro* in HaCat cells. 167 mg of curcumin and 167 mg of resveratrol were weighed in a 50 ml Falcon test tube; 20 ml of DMEM medium were added to it.

After mixing the aforesaid components, the sample was filtered with 0.45 and 0.22 µm filters in order to remove the insoluble fraction.

*Multiwell* plates with six wells were used for seeding 100,000 cells in each of them, which were left to grow until reaching a confluence of 90%. Subsequently, the cells were incubated for 120 minutes at 37°C with enriched medium as previously described. At the end of the treatment, the medium was sampled and, in order to remove any precipitates, the cells were washed three times with 1 ml of PBS which was then added to the medium just sampled. Such sample was filtered and analysed through chromatography analysis according to the HPLC-DAD chromatography method described above in Example 1a to determine the concentrations of curcumin and resveratrol in the DMEM medium after contact with the cells.

The cells were detached mechanically using a scraper in 2 ml of PBS and transferred into a 15 ml Falcon test tube for being centrifuged at 1500 rpm for 5 minutes. Once the supernatant had been removed, the cell pellet was re-suspended in 500 µl of 96°ethanol in order to break the cell membranes and allow the release and solubilisation of any curcumin and resveratrol absorbed by them. The cell residues were removed through centrifugation at 6000 rpm for 10 minutes. The sample thus obtained was filtered and analysed through chromatography analysis according to the HPLC-DAD chromatography method described above in Example 1a to evaluate the quantities of curcumin and resveratrol absorbed in the same operating conditions previously described. The results obtained, reported in Table 4, were expressed as a mean of the technical triplicates. The standard deviation, not reported, is less than 5%.

**Table 4: Results obtained from the chromatography analysis of the DMEM medium with curcumin and resveratrol added ("DMEM medium" line) and of the cell pellet after treatment ("Cell pellet" line)**

| | Curcumin (µg/ml) | Resveratrol (µg/ml) |
|---|---|---|
| DMEM medium | 0 | 31.79 |
| Cell pellet | 0 | 0 |

The results in terms of solubilisation of the DMEM medium were substantially in line with those obtained in example 1 with the SSF: curcumin was shown to be completely insoluble, whereas for resveratrol - also insoluble in SSF - a concentration equal to only 31.79 µg/ml was found.

Instead, the results obtained from the cell pellet analysis highlighted the absence of absorption of the two compounds by the cells.

### Example 2b - Evaluation of the oral bioavailability of curcumin and resveratrol in the presence of matrix in vitro in HaCat cells, in the presence of a chocolate matrix comprising cocoa, ethanol and maltitol

For the purpose of evaluating the oral bioavailability of curcumin and resveratrol in HaCat cells in the presence of the chocolate matrix, of maltitol and ethanol, 200 mg of curcumin, 200 mg of resveratrol, 5.4 g of maltitol and 12 g of Venchi dark chocolate were weighed. The chocolate was dissolved in a beaker in bain-marie at 50°C and the components previously listed and 799.2 µl of 96° ethanol were added. The sample, thus prepared, was incubated at -80°C for 30 minutes and then reduced into smaller pieces. Three extractions were performed with 30 ml of hexane and, in order to remove the lipid component of the chocolate matrix, the emulsion was centrifuged at 6000 rpm for 10 minutes and the cell pellet obtained was dried in a nitrogen atmosphere.

Subsequently, the cell pellet was re-suspended in 24 ml of DMEM medium and the sample was centrifuged at 6000 rpm for 10 minutes to remove the insoluble part. The supernatant was, finally, filtered with filters at 0.45 and 0.22 µm. The quantities of the compounds added to the medium and were selected on the basis of the first experimental phase.

The cells were incubated with the medium thus prepared for 120 minutes at 37°C. After the end of the treatment, the cells were processed as described in the first test of the second experimental phase. The chromatography analyses were performed using the HPLC-DAD chromatography method described above in Example 1a.

The results obtained, reported in Table 5, were expressed as a mean of the technical triplicates. The standard deviation, not reported, is less than 5%.

**Table 5: Results obtained from the chromatography analysis of the DMEM medium with chocolate matrix, curcumin, resveratrol, maltitol and ethanol added ("DMEM medium" line) and of the cell pellet after treatment ("Cell pellet" line)**

| | Curcumin (µg/ml) | Resveratrol (µg/ml) |
|---|---|---|
| DMEM medium | 6.58 | 124.82 |
| Cell pellet | 4.50 | 3.66 |

The results in terms of solubilisation show that curcumin and resveratrol have a concentration of 6.58 µg/ml and 124.82 µg/ml, respectively, in the DMEM medium, demonstrating that the chocolate matrix significantly improves the solubility of the two compounds. The analysis of the cell pellets allowed the concentration of curcumin and resveratrol in 4.50 µg/ml and 3.66 µg/ml to be quantified, demonstrating that the chocolate matrix comprising cocoa, ethanol and maltitol promotes the absorption of the two compounds by the cells in an aqueous means.

On the basis of the results obtained, it was possible to highlight that the chocolate matrix comprising cocoa, ethanol and maltitol therefore has a solubilising effect towards plant derivatives such as resveratrol and curcumin in an aqueous means, also in the presence of a means simulating salivary fluid. Furthermore, it is possible to highlight that said matrix promotes not only the solubility but also the cell absorption of curcumin and resveratrol in an aqueous environment.

On the basis of this evidence, it is possible to conclude that the chocolate matrix comprising cocoa, ethanol and maltitol also leads to an increase in the oral bioavailability of said plant derivative, thus overcoming the limits of the prior art regarding their effectiveness at nutritional and physiological level.

## Claims

1. A chocolate matrix comprising cocoa, ethanol, maltitol and at least one plant derivative selected from the group consisting of: curcumin and resveratrol.

2. The chocolate matrix according to claim 1, comprising ethanol in an amount from 0.003 to 0.009% by weight, with respect to the total weight of said matrix.

3. The chocolate matrix according to claim 1 or 2, comprising maltitol in an amount from 30 to 55% by weight, with respect to the total weight of said matrix.

4. The chocolate matrix according to anyone of claims 1-3, comprising the at least one plant derivative in an amount from 1 to 2% by weight, with respect to the total weight of said matrix.

5. The chocolate matrix according to anyone of claims 1-4, with respect to the total weight of said matrix, from 40 to 65% by weight of cocoa, from 0.003 to 0.009% by weight of ethanol, from 30 to 55% by weight maltitol, and from 1 to 2% by weight of at least one plant derivative selected from the group consisting of: curcumin and resveratrol.

6. A nutraceutical product comprising the chocolate matrix according to anyone of claims 1-5 and at least one nutraceutically suitable ingredient.

7. The nutraceutical product according to claim 6, for use as a food supplement or as a functional food.

8. The nutraceutical product according to claim 6 or 7, in the form of a capsule, granulate, pill, candy, tablet, paste, cream.

9. A nutraceutical kit for use as a food supplement or as a functional food comprising at least one nutraceutical product according to anyone of claims 6-8 and instructions for the user including information related to the use of the nutraceutical product.

10. A confectionery product comprising the chocolate matrix according to anyone of claims 1-5 and at least one suitable food additive.

## Patentansprüche

1. Schokoladenmatrix, die Kakao, Ethanol, Maltitol und mindestens ein pflanzliches Derivat enthält, das aus der Gruppe ausgewählt ist, die aus Curcumin und Resveratrol besteht.

2. Schokoladenmatrix nach Anspruch 1, umfassend Ethanol in einer Menge von 0,003 bis 0,009 Gew.-%, bezogen auf das Gesamtgewicht der Matrix.

3. Schokoladenmatrix nach Anspruch 1 oder 2, die Maltitol in einer Menge von 30 bis 55 Gew.-%, bezogen auf das Gesamtgewicht der Matrix, enthält.

4. Schokoladenmatrix nach einem der Ansprüche 1 bis 3, umfassend das mindestens eine Pflanzenderivat in einer Menge von 1 bis 2 Gew.-%, bezogen auf das Gesamtgewicht der Matrix.

5. Schokoladenmatrix nach einem der Ansprüche 1 bis 4, bezogen auf das Gesamtgewicht der Matrix, 40 bis 65 Gew.-% Kakao, 0,003 bis 0,009 Gew.-% Ethanol, 30 bis 55 Gew.-% Maltit und 1 bis 2 Gew.-% mindestens eines pflanzlichen Derivats, ausgewählt aus der Gruppe bestehend aus: Curcumin und Resveratrol.

6. Nutrazeutisches Produkt, umfassend die Schokoladenmatrix nach einem der Ansprüche 1 bis 5 und mindestens eine nutrazeutisch geeignete Zutat.

7. Nutrazeutisches Produkt nach Anspruch 6 zur Verwendung als Nahrungsergänzungsmittel oder als funktionelles Lebensmittel.

8. Nutrazeutisches Produkt nach Anspruch 6 oder 7, in Form einer Kapsel, eines Granulats, einer Pille, eines Bonbons, einer Tablette, einer Paste oder einer Creme.

9. Nutrazeutisches Kit zur Verwendung als Nahrungsergänzungsmittel oder als funktionelles Nahrungsmittel, umfassend mindestens ein nutrazeutisches Produkt nach einem der Ansprüche 6 bis 8 und Anweisungen für den Benutzer, die Informationen bezüglich der Verwendung des nutrazeutischen Produkts enthalten.

10. Süßwarenprodukt, umfassend die Schokoladenmatrix nach einem der Ansprüche 1-5 und mindestens einen geeigneten Lebensmittelzusatzstoff.

## Revendications

1. Matrice de chocolat comprenant du cacao, de l'éthanol, du maltitol et au moins un dérivé végétal choisi dans le groupe composé par : la curcumine et le resvératrol.

2. Matrice de chocolat selon la revendication 1, comprenant de l'éthanol en une quantité de 0,003 à 0,009% en poids, par rapport au poids total de ladite matrice.

3. Matrice de chocolat selon la revendication 1 ou 2, comprenant du maltitol en une quantité de 30 à 55% en poids, par rapport au poids total de ladite matrice.

4. Matrice de chocolat selon l'une quelconque des revendications 1 à 3, comprenant l'au moins un dérivé végétal en une quantité de 1 à 2% en poids, par rapport au poids total de ladite matrice.

5. Matrice de chocolat selon l'une quelconque des revendications 1 à 4, par rapport au poids total de ladite matrice, de 40 à 65% en poids de cacao, de 0,003 à 0,009% en poids d'éthanol, de 30 à 55% en poids de maltitol, et de 1 à 2% en poids d'au moins un dérivé végétal choisi dans le groupe composé par : la curcumine et le resvératrol.

6. Produit nutraceutique comprenant la matrice de chocolat selon l'une quelconque des revendications 1 à 5 et au moins un ingrédient convenant aux nutraceutiques.

7. Produit nutraceutique selon la revendication 6, destiné à être utilisé comme complément alimentaire ou comme aliment fonctionnel.

8. Produit nutraceutique selon la revendication 6 ou 7, sous forme de capsule, granulé, pilule, bonbon, comprimé, pâte, crème.

9. Kit nutraceutique destiné à être utilisé comme complément alimentaire ou comme aliment fonctionnel comprenant au moins un produit nutraceutique selon l'une quelconque des revendications 6 à 8 et des instructions pour l'utilisateur comprenant des informations relatives à l'utilisation du produit nutraceutique.

10. Produit de confiserie comprenant la matrice de chocolat selon l'une quelconque des revendications 1 à 5 et au moins un additif alimentaire approprié.
